# EUROPEAN PATENT APPLICATION

(11) **EP 4 193 908 A1**
(43) Date of publication of application: **14.06.2023**
(21) Application number: 21213064.5
(22) Date of filing: 08.12.2021
(51) Int. Cl.: A61B 5/00, A61B 5/06, A61B 5/107

(54) **IMPROVING MAPPING OF AN ANATOMICAL CAVITY AND/OR LOCATION TRACKING IN THE ANATOMICAL CAVITY**

(71) Applicant: Koninklijke Philips N.V., 5656 AG Eindhoven (NL)
(72) Inventor: HENDRIKS, Bernardus Hendrikus Wilhelmus, Eindhoven (NL); WILDEBOER, Rogier Rudolf, Eindhoven (NL); KAHLMAN, Josephus Arnoldus Johannes Maria, Eindhoven (NL); SPLIETHOFF, Jarich Willem, Eindhoven (NL); GIJSBERS, Gerardus Henricus Maria, Eindhoven (NL); SCHÄFER, Dirk, Eindhoven (NL); KOLEN, Alexander, Eindhoven (NL); RUETTEN, Walter, Eindhoven (NL)
(74) Representative: Philips Intellectual Property & Standards

(57) **Abstract**

A mechanism for generating feedback information for aiding in improved performance of tracking the location of an interventional device, where such tracking is performed using one or more electric, electromagnetic and/or magnetic fields generated by one or more field generators on the surface of the subject. A spatial relationship between field generators on a surface of the subject and one or more anatomical structures of the subject is determined from non-invasive imaging data. This spatial relationship is used to generate the feedback information for calibrating a mapping or location tracking system and/or for repositioning the field generators.

## Description

### FIELD OF THE INVENTION

The present invention relates to the field of medical subject analysis, and in particular, to the tracking of interventional devices.

### BACKGROUND OF THE INVENTION

There is an increasing interest in the (computer) modelling of anatomical structures of a subject, to provide a visual representation of the anatomical structure. This aids analysis of the anatomical structure, e.g. during the course of a medical procedure or diagnosis.

One area of particular interest is in mapping of an anatomical structure (such as the heart), in which an interventional catheter is inserted into the anatomical cavity of the anatomical structure and navigation and/or mapping systems are used to localize the catheter, which can then be used to generate an anatomical model of the anatomical cavity or structure. One example, of a suitable mapping system is the EPD Solutions navigation system provided by Philips^{®}, named the KODEX-EPD^{®} system, which exploits (di)electric field sensing for 3D localization, allowing radiation-free navigation in an anatomical cavity, e.g. inside the heart.

Some mapping techniques, such as those employed by the KODEX-EPD system, are based on electrical fields between three pairs of electrode-patches that represent the x-, y-, and z-dimension. During the procedure, a real-time voltage-to-position function is formed. This function is continuously updated based on the measurements coming in. Once sufficiently updated, the voltage-to-position function enables the system to reliably display the spatial movement of the catheter.

At the same time, the stored catheter measurements are used to anatomically reconstruct an anatomical model of the anatomical element (e.g. the heart). The catheter position can then be determined with respect to the anatomical model, allowing accurate navigation towards and around anatomical features.

Other mapping techniques may use magnetic or electromagnetic fields instead of the electrical fields to achieve a similar effect.

There is a clear desire for improved mapping of an anatomical structure and/or improved tracking of an interventional device within the anatomical structure.

### SUMMARY OF THE INVENTION

The invention is defined by the claims.

According to examples in accordance with an aspect of the invention, there is provided a processing system for generating feedback information responsive to positions of one or more field generators on the surface of a subject, wherein the one or more field generators are usable for generating one or more electric, magnetic and/or electromagnetic fields for tracking the location of an interventional device in the subject.

The processing system comprises an input interface configured to receive: non-invasive imaging data of a subject, wherein the non-invasive imaging data contains a representation of one or more anatomical structures of the subject and the one or more field generators positioned on the surface of the subject. The non-invasive imaging data is generated through a non-invasive imaging procedure of the subject and the one or more field generators.

The processing system also comprises a processing unit communicatively coupled to the input interface and configured to: process the non-invasive imaging data to determine a spatial relationship between the one or more anatomical structures of the subject and the one or more field generators; and generate the feedback information responsive to the determined spatial relationship.

The feedback information comprises: calibration information for an anatomical mapping system and/or location tracking system that uses the one or more electric, magnetic and/or electromagnetic fields generated using the one or more field generators to perform an anatomical mapping or location tracking operation respectively; and/or repositioning information indicating any recommended positional changes for the one or more field generators to realize a desired spatial relationship between the one or more anatomical structures of the subject and the one or more field generators;

The processing system may comprise an output interface configured to provide the feedback information.

Embodiments propose approaches for generating feedback about a spatial relationship between one or more field generators and one or more anatomical structures in a subject. It has been recognized that this would provide valuable information for calibrating a tracking or mapping system that relies upon fields generated by the field generator(s) and/or for repositioning the field generator(s) to improve an accuracy or reliability of a tracking or mapping system. Non-invasive imaging data is used to establish this spatial relationship, which reduces risk to the subject.

The disclosed approach accurately and reliably, establishes the spatial relationship any field generators and internal anatomical structures, since the non-invasive imaging data will accurately identify this relationship. In particular, the imaging data may be captured at a same time, to provide accurate temporal registration between the anatomical structure(s) and the field generator(s).

Knowing the position of the anatomical structure(s) with respect to the field generator(s) facilitates identifying of any potential influence on an electric/magnetic/electromagnetic field inside the body produced by the field generator(s). This relationship is therefore important for calibrating measurements of the fields (e.g. by an interventional device).

Moreover, knowing the position of the anatomical structure(s) with respect to the field generator facilitates repositioning of the field generators so that any generated fields will intersect or pass through a desired anatomical region (e.g. a region to be imaged using an interventional device that makes use of the generated fields).

The one or more anatomical structures may comprise only internal anatomical structures of the subject, e.g. structures not visible within a visible light spectrum through unbroken skin.

In at least one embodiment, the non-invasive imaging data comprises penetrative imaging data of the subject, for identifying the one or more anatomical structures, and non-penetrative imaging data of a surface of the subject, for identifying the one or more field generators. Examples of penetrative imaging data include X-ray, CT, ultrasound and MR imaging data.

The non-invasive imaging data may be generated by an imaging system that generates imaging data using two or more detector arrangements having a known spatial relationship with respect to one another. In this way, the spatial relationship of the anatomical structure(s) and the field generator(s) can be readily determined, as the co-ordinate systems of the detector arrangements are directly registered with respect to one another.

Optionally, the one or more anatomical structures of the object comprise one or more anatomical structures that would distort any electric, magnetic and/or electromagnetic fields generated by the one or more field generators. Suitable examples include any ill-conducting anatomical structures, such as the lungs (e.g. due to the presence of air in the lungs) and other anatomical structures.

In some examples, the one or more anatomical structures comprises at least one desired anatomical structure for insertion of an interventional device.

In some examples, the feedback information contains repositioning information that identifies recommended changes in position for the one or more field generators such that, when repositioned, an electric, magnetic and/or electromagnetic fields generated by the one or more field generators would intersect the at least one desired anatomical structure.

Optionally, the calibration information comprises the determined spatial relationship between the one or more anatomical structures of the subject and the one or more field generators.

In at least one embodiment, the processing unit is further configured to process the non-invasive imaging data to identify a size and/or shape of the one or more anatomical structures; and the calibration information further includes information on the identified size and/or shape of the one or more anatomical structures.

There is also proposed a mapping system comprising: a processing system as herein described; and an anatomical mapping system comprising: an input module configured to receive: one or more electrical responses, each electrical response being a response of a field-sensitive sensor, positioned on or in an interventional device, to any electric, magnetic and/or electromagnetic fields generated by the one or more field generators as the interventional device moves within an anatomical cavity of the subject; and the feedback information generated by the processing unit of the processing system; a processing module, communicatively coupled to the input module, configured to process the one or more electrical responses to generate an anatomical map or model of the anatomical cavity, wherein the processing module calibrates the one or more electrical responses using the spatial relationship between the one or more anatomical structures of the subject and the one or more field generators contained in the feedback information;.

The anatomical mapping system may comprise an output module configured to provide the anatomical map or model.

In some examples, each electrical response is a voltage measured by a voltage sensor positioned on or in the interventional device, and the processing module is configured to generate the anatomical map or model of the anatomical cavity using a voltage-to-position function.

There is also proposed a repositioning feedback system comprising any herein described processing system, wherein the feedback information comprises at least the repositioning information; and a user interface configured to display a visual representation of the repositioning information.

There is also proposed a computer-implemented method for generating feedback information responsive to positions of one or more field generators on the surface of a subject, wherein the one or more field generators are usable for generating one or more electric, magnetic and/or electromagnetic fields for tracking the location of an interventional device in the subject.

The computer-implemented method comprises: receiving, at an input interface: non-invasive imaging data of a subject, wherein the non-invasive imaging data contains a representation of one or more anatomical structures of the subject and the one or more field generators positioned on the surface of the subject, wherein the non-invasive imaging data is generated through a non-invasive imaging procedure of the subject and the one or more field generators are usable for generating one or more electric, magnetic and/or electromagnetic fields for tracking the location of an interventional device in the subject.

The computer-implemented method further comprises processing, using a processing unit, the non-invasive imaging data to determine a spatial relationship between the one or more anatomical structures of the subject and the one or more field generators; and generating, using the processing unit, the feedback information responsive to the determined spatial relationship.

The feedback information comprises: calibration information for an anatomical mapping system and/or location tracking system that uses the one or more electric, magnetic and/or electromagnetic fields generated using the one or more field generators to perform an anatomical mapping or location tracking operation respectively; and/or repositioning information indicating any recommended positional changes for the one or more field generators to realize a desired spatial relationship between the one or more anatomical structures of the subject and the one or more field generators.

The computer-implemented method may further comprise a step of providing, at an output interface, the feedback information.

Any herein described method may be adapted to perform operations performed by any herein described processing system, and vice versa.

There is also proposed a computer program product comprising computer program code means which, when executed on a computing device having a processing system, cause the processing system to perform all of the steps of any herein described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates positions for a set of field generators positioned on a subject;
Fig. 2 illustrates a co-ordinate system for defining a direction of fields generated by the field generators;
Fig. 3 illustrates a system for tracking a field-sensitive sensor and/or generating an anatomical model of an anatomical structure;
Fig. 4 illustrates an approach for reconstructing an anatomical model from a point cloud;
Fig. 5 illustrates a processing system;
Fig. 6 illustrates a system;
Fig. 7 is a flowchart illustrating a method; and
Fig. 8 is a block diagram illustrating a processing system.

### DETAILED DESCRIPTION OF THE EMBODIMENTS

The invention will be described with reference to the Figs..

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs. are merely schematic and are not drawn to scale. It should also be understood that the same reference numerals are used throughout the Figs. to indicate the same or similar parts.

The invention provides a mechanism for generating feedback information for aiding in improved performance of tracking the location of an interventional device, where such tracking is performed using one or more electric, electromagnetic and/or magnetic fields generated by one or more field generators on the surface of the subject. A spatial relationship between field generators on a surface of the subject and one or more anatomical structures of the subject is determined from non-invasive imaging data. This spatial relationship is used to generate the feedback information for calibrating a mapping or location tracking system and/or for repositioning the field generators.

Embodiments are based on the realization that improved accuracy in calibrating or repositioning of field generators (e.g. to achieve better field properties) can be achieved by determining a spatial relationship between anatomical structure and field generators. In particular, knowledge of this spatial relationship can allow for predictions about field properties within the subject to be determined, e.g. areas of probable distortion identified, to allow for more accurate calibration (e.g. to take account of such areas) or repositioning of field generators (e.g. to reduce or avoid such areas). This, in turn, facilitates more accurate tracking of the location of field-sensitive sensors, and devices containing such sensors, within the subject, and consequently improved imaging or mapping using such tracked locations.

Embodiments may be employed in any suitable clinical environment in which the tracking of a location of an interventional device, such as a catheter, is performed.

Thus, the present invention provides approaches for providing feedback on the positions of field generators to effectively calibrate for electric, magnetic or electromagnetic fields that are not in desired positions or to provide repositioning information for the field generators to achieve a desired orientation and/or spatial relationship between any electric, magnetic or electromagnetic fields.

For the purposes of contextual understanding, the principle and purpose of inducing fields within a subject (i.e. an individual, such as a person or animal) is described.

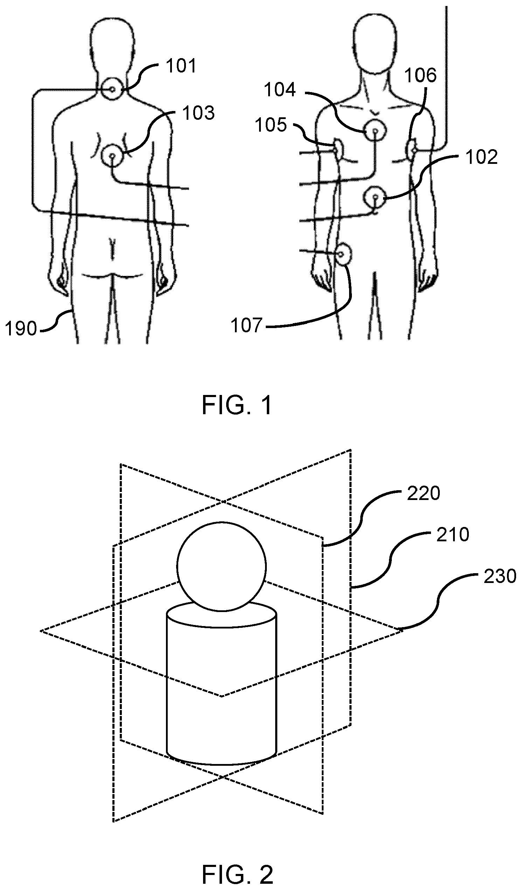

Fig. 1 illustrates positions for a set of field generators positioned on a subject 190. The field generators are configured to generate a plurality of crossing electric, magnetic or electromagnetic fields ("fields").

The illustrated set of field generators comprises a first field generator 101, a second field generator 102, a third field generator 103, a fourth field generator 104, a fifth field generator 105 and a sixth field generator 106. The illustrated set further comprises a reference sensor 107, which can act as a "ground" for defining a base/background level of field activity in the subject.

An electric signal provided to each field generator is controlled to thereby define crossing fields therebetween. In particular, the first 101 and second 102 field generators form a first pair of field generators ("field generator pair"), and signals provided to the first field generator pair can be controlled to induce a first field therebetween. The third 103 and fourth 104 field generators form a second pair of field generators, and signals provided to the second field generator pair can be controlled to induce a second field therebetween. The fifth 105 and sixth 106 field generators form a third pair of field generators, and signals provided to the third field generator pair can be controlled to induce a third field therebetween.

As an example, each field generator may be an electrode, and a voltage or current provided to each electrode may control a strength and/or frequency of an electric field generated using that electrode. As another example, each field generator may be an electromagnetic, in which an electric signal provided to each electrode controls a strength and/or frequency of a magnetic field generated using the electromagnet.

Electric signals provided to each field generator, and in particular to each field generator pair, can control the frequency and/or magnitude/strength of the crossing fields. The electric signals supplied to the field generators may be controlled by a field control system (not illustrated in Fig. 1).

The crossing electric fields are usable to track or identify a location of any field-sensitive sensor positioned within the (overlap of the) crossing fields. For instance, where the crossing fields are crossing electric fields, a position of an electrode within the crossing electric fields can be determined. Where the crossing fields are crossing magnetic fields, a position of a magnetically-responsive sensor (e.g. a Hall sensor) within the crossing magnetic fields could be tracked or determined.

In particular, a response of a field-responsive sensor to each field will change as a position within the field changes (e.g. a distance from a source/sink of the field changes). The response of a field-responsive sensor to the crossing fields can be mapped or associated with a particular position within a multidimensional co-ordinate system using a mapping function.

In other words, a response (which can be alternatively labelled a "measurement") of the field-responsive sensor to the crossing fields can be processed to determine a relative position of the field-responsive sensor (e.g. and therefore of any interventional device comprising the sensor) within an anatomical cavity of the subject.

A mapping function may be used to map a sampled response of a field-responsive sensor to a position within a multidimensional co-ordinate space (i.e. to a position in space, namely a Euclidian/Cartesian co-ordinate space).

The mapping function may be required because the fields are non-linear, meaning that a position defined using a response alone (i.e. without mapping) does not necessarily relate to a position in actual space. The coefficients (values of parameters) of the mapping function represent scaling factors and/or local distortions to map a position in the field space to a predicted position in a "true" space (or R-space).

As a very simple example, where the crossing fields are electric fields, the mapping function may map an electrical response, such as a voltage, Vx to a position Rx. One example of a mapping function takes the form of Rx = a^{∗}Vx+b. The mapping function thereby contains coefficients or parameters a and b, for which values need to be acquired. Other, more complex mapping functions, may be used instead.

WO2018130974 describes one example approach for generating a mapping function. In this document, voltage measurements obtained by two electrodes on a catheter are processed to generate a mapping function that transforms electrical potential to a position in space. In particular, a known physical distance between the two electrodes (in combination with the paired voltage measurements) is used to determine the mapping function. The use of the known physical distance can (at least partially) facilitate correction for any distortion of the crossing fields. However the greater the distortion of the crossing fields, the less accurate reconstruction will be. Later described embodiments aim to at least partially resolve or address this issue.

Other approaches for generate a mapping/transfer function would be apparent to the skilled person.

Each field may be controlled to have a particular/different frequency. This facilitates determination of the relative position of a field-sensitive sensor with respect to each individual field, by assessing the response of the field-responsive sensor to the particular frequency of the field. This information can be used to effectively identify or predict the position of the field-responsive sensor with respect to a co-ordinate system defined by the crossing fields.

One or more of the field generator pairs may be omitted, e.g. if only two crossing fields are desired (e.g. for performing a 2-dimensional tracking process). It may be possible to replace one or more of the pairs of field generators with a single field generator (for each pair). This is possible if generation of the field can be performed using a single field generator, e.g. a single electromagnet may generate a magnetic field.

Fig. 2 schematically illustrates a co-ordinate system for defining a direction of fields generated by the field generators. The co-ordinate system defines three cardinal planes 210, 220, 230. The crossing fields may, for instance, be optimally positioned when the direction of each field is parallel to a respective cardinal place 210, 220, 230.

The intent of the crossing fields, e.g. such as those generated using the set illustrated by Fig. 1, is to facilitate identification of the position of one or more field-sensitive sensors with respect to a co-ordinate system, such as that illustrated by Fig. 2.

A more complete example of how to track the position of a field-sensitive sensor with respect to crossing fields (i.e. within the subject), and optionally how to further exploit this information, e.g. for the construction of an anatomical model of an anatomical cavity of the subject, is hereafter described for the purposes of contextual understanding.

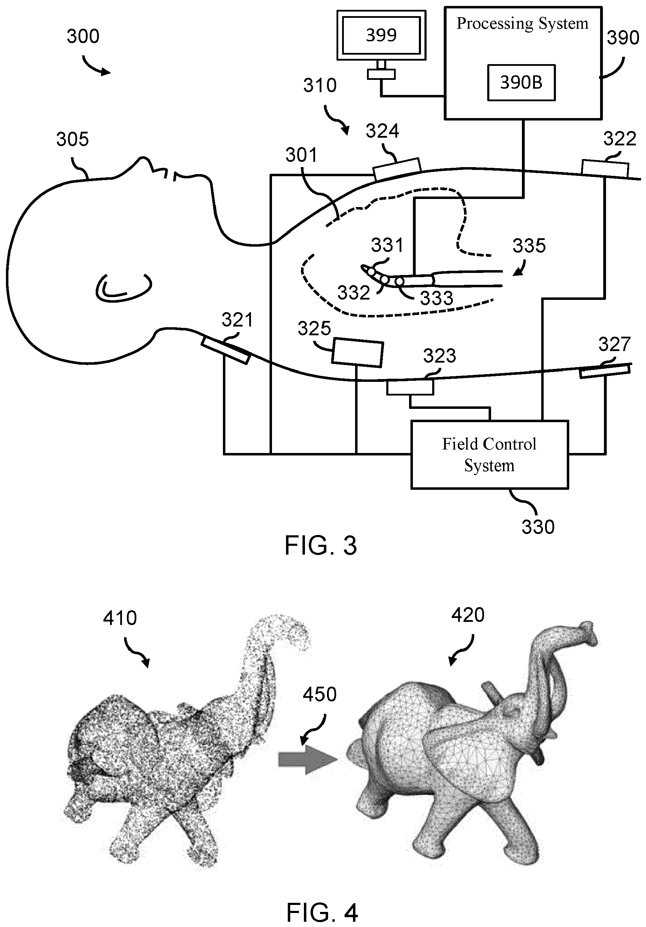

Fig. 3 conceptually illustrates a processing system 300 for generating a mapping function for predicting the position of a field-sensitive sensor within an anatomical cavity 301 of a subject 305 (e.g. blood vessels, gastrointestinal tract, the cardiac system and so on).

The processing system 300 may further generate an anatomical model of an anatomical cavity within a subject. In particular examples, the processing system 300 may be configured to generate an anatomical model (of an anatomical cavity, such as a blood vessel and/or chamber) using an imaging process.

The processing system 300 comprises an exemplary field generating arrangement 310 and a processing system 390.

The processing system 390 is configured to generate a mapping function for mapping responses of field-sensitive sensors to a position within a multidimensional co-ordinate space (i.e. to a position in a Euclidian/Cartesian co-ordinate space). Thus, the processing system 390 acts as a mapping function generator.

The processing system 390 may also contain a mapping processor 390B, although this may be alternatively positioned externally to the processing system 390.

In the illustrated example, the field generating arrangement 310 comprises a set of field generators 321, 322, 323, 324, 325, 327 positioned externally with respect to the subject 305.

The set 310 of field generators may comprise a plurality of field generator pairs, which are preferably angled with respect to one another, so that any fields generated by the pairs are angled with respect to one another. These pairs may comprise a first field generator pair (formed of a first 321 and second 322 field generator), a second field generator pair (formed of a third 323 and fourth 324 field generator) and a third field generator pair (formed of a fifth 325 and sixth (not visible) field generator). One of more of these field generator pairs may be omitted. The set of field generators may also comprise a reference sensor 327. The field generators are placeable in a similar manner to the set of field generators illustrated in Fig. 1.

As previously explained, it may be possible to replace any field generator pair with a single field generator.

The field generating arrangement 310 also comprises a field control system 330, which is adapted to control (characteristics of) the fields generated by the field generators, e.g. by controlling an electric signal (e.g. voltage and/or current) supplied to each field generator. In some examples, this may form part of the processing system 390. The field generating arrangement 310 is thereby configured to generate and control multiple (here: three) crossing (intra-body) fields using the field generators.

In particular examples, each field generator pair may be appropriately controlled to induce a field between each field generator pair. Thus, where there are three field generator pairs, three field generator fields may be generated. Of course, any of the field generator pairs may be replaced with an appropriately controlled single field generator.

Preferably, the frequency of each generated field is controlled to be different, to facilitate increased ease in identifying a relative location of a field-sensitive arrangement positioned within the crossing fields.

The generated fields can be used to define or establish a relative location of a field-sensitive sensor positioned within the crossing fields. In particular, as previously explained, the response of a field-sensitive sensor to the fields changes as the relative position of the sensor moves about the subject. This is at least partly because the induced fields' distribution is inherently inhomogeneous due to the different dielectric properties and absorption rates (related to conductivity) of the interrogated tissues and/or natural attenuation of field strength with distance from a field generator. The principle of crossing fields thereby facilitates tracking of the relative position of the field-sensitive sensor using a processing system 390 that monitors a response of any field-sensitive sensors to the crossing fields, e.g. by mapping the response of a field-sensitive sensor to a relative position within the subject or using a suitable mapping/transfer function, to determine the relative position(s) of the field sensitive-sensor(s).

Approaches for monitoring the location of a field-sensitive sensor, when the fields are magnetic fields, are established in the art, for instance as described by, inter alia, WO 2017/201288 A1, US 2021/052191 A1 and WO 2012/150567 A1. Any of these approaches, or others established in the art, could be employed for use in the present disclosure.

Approaches for monitoring the location of a field-sensitive sensor, when the fields are crossing electric fields, are similarly known in the art, as disclosed by the European Patent Applications EP 0775466 A2, EP 3568068 A1 and EP 3607879 A1. Any of these approaches, or others established in the art, could be employed for use in the present disclosure.

Once the relative locations of the catheter and/or one or more sensors on the catheter have been established, it is possible to construct an anatomical model of an anatomical cavity (i.e. a cavity in which the interventional device is able to move). This process is performed by the mapping system 390B.

Broadly, the mapping system 390B may build an anatomical model of an anatomical cavity 301 (e.g. a chamber, vessel or void) within a subject by processing a cloud of points. Each point represents a position of the catheter or sensor in a defined space. More specifically, a reconstruction algorithm is used to generate an anatomical model of the (investigated part of the) anatomical cavity. The anatomical model may, for example, be a 3D surface that depicts or models the (known bounds of) the anatomical cavity.

The process of reconstructing an anatomical model from a point cloud is conceptually illustrated in Fig. 4, which demonstrates a process 450 in which a cloud of points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

The skilled person will appreciate that the derived mapping/transfer function can also be used to track a location of one or more field-sensitive sensors with respect to a constructed anatomical model. This could facilitate the generation and display of an anatomical model and an indicator of a current location/position of an interventional device 335 mounting or carrying one or more field sensitive sensors 331, 332, 333 with respect to the anatomical model. Of course, a visual representation of any generated anatomical model and/or determined position may be generated and provided at an output display 399.

The present invention relates to approaches for determining a relative position of field generators and providing suitable feedback information for calibrating a mapping/transfer function and/or repositioning the field generators. In particular, a spatial relationship between the field generator(s) and anatomical structures is determined, and used to provide the feedback information.

In particular, it is herein recognized that it would be preferable for crossing fields to intersect at a desired region and/or to be substantially orthogonal to one another. This facilitates improved accuracy in tracking or determining the location of any field-sensitive sensors (and devices comprising any such sensors) as well as subsequent mapping that makes use of such locations.

This disclosure recognizes that a spatial relationship between the field generator(s) and anatomical structures can be used (e.g. as a proxy) to represent the relationship between the crossing electric fields and the designed regions. Embodiments make use of this recognition to produce feedback information, for calibrating a mapping/location function or repositioning field generators, to improve the accuracy of any mapping or location approaches.

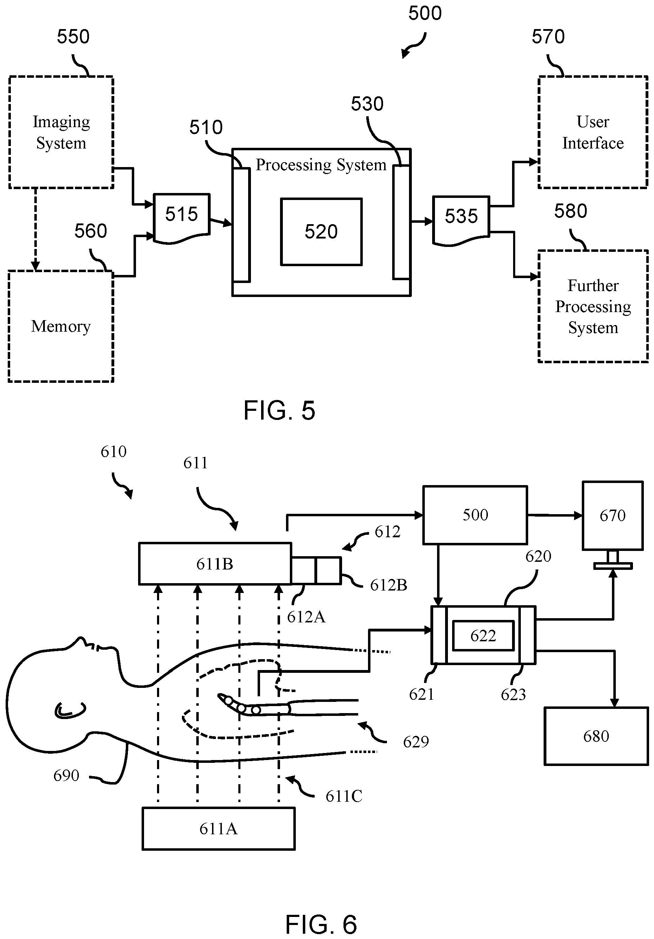

Fig. 5 conceptually illustrates a processing system 500 according to an embodiment of the invention. The processing system 500 comprises an input interface 510, a processing unit 520 and an optional output interface 530. The processing unit 520 communicatively coupled to the input interface 510 and, if present, the output interface 530.

The input interface 510 is configured to receive non-invasive imaging data 515 of a subject. The non-invasive imaging data 515 contains a representation of one or more anatomical structures of the subject and the one or more field generators positioned on the surface of the subject. The non-invasive imaging data is generated through a non-invasive imaging procedure of the subject and the one or more field generators.

The one or more anatomical structures of the subject are preferable internal structures of the subject, e.g. organs, vessels, bones or internal tissue.

The non-invasive imaging data may, for instance, be obtained from an imaging system 550 or memory/storage unit 560, which may in turn receive or store data from the imaging system 550.

Different forms of non-invasive imaging data may be used to separately track the anatomical structure(s) and the field generator(s). For instance, penetrative imaging data (such as X-ray image data, CT image data, ultrasound image data and/or MR imaging data) could be used to identify anatomical structure(s). Non-penetrative imaging data, e.g. data obtained using one or more cameras, could be used to track/identify the field generator(s).

The processing unit 520 is configured to process the non-invasive imaging data to determine a spatial relationship between the one or more anatomical structures of the subject and the one or more field generators.

A spatial relationship may refer to any one or more properties that indicates or represents a relative position and/or orientation of each field generator with respect to each anatomical structure. Thus, a spatial relationship may comprise one or more measures of distance and/or (difference in) orientation between each field generator and each anatomical structure.

For instance, a spatial relationship may contain a measure of distance between each field generator and each anatomical structure. A spatial relationship may contain a vector defining a direction and distance between each field generator and each anatomical structure. A spatial relationship may contain a difference in orientation between each field generator and each anatomical structure. Any combination of these potential measures may form part of the spatial relationship.

If the non-invasive imaging data comprises a single, uniform set of image data containing a representation of both the anatomical structure(s) and the field generator(s), then determining a spatial relationship is fairly straightforward, e.g. by directly determining distances and/or orientations using the uniform set of image data. Suitable examples of uniform sets of image data include CT image data, e.g. in which the position of field generators could be identified as highly contrasting structures (e.g. if they are made of metal or other similar materials).

Approaches for processing image data to identify the location of anatomical structures are well established in the art, e.g. using any known image segmentation technique. Such approaches may be adapted to identify any field generators in the image data, e.g. by identifying a shape of the field generator(s).

It has previously been explained how different forms of non-invasive imaging data may be used to separately track the anatomical structure(s) and the field generator(s). In particular, penetrative imaging data may be used to identify the one or more anatomical structures, and non-penetrative imaging data of a surface of the subject may be used to identify any field generators.

In these examples, the penetrative imaging data may be obtained by a first imaging system and the non-penetrative imaging data may be generated by a second imaging system. The cameras and/or detectors of the first and second imaging systems may have a known spatial relationship to one another. In other words, the co-ordinate systems of the first and second imaging systems may be directly linked to one another. This known spatial relationship facilitates ease of identifying a spatial relationship between the anatomical structure(s) and the field generator(s).

If used, the penetrative and non-penetrative information should be obtained across a period of time, so that there is a direct and accurate spatial relationship between the two.

Of course, in other examples, image data generated by a first and second imaging system may be separately registered to one another. For instance, an outline of the subject may be identified in the penetrative imaging data and the non-penetrative imaging data in order to register the two types of imaging data with respect to one another.

To improve an ease of identifying any field generator(s) in the non-penetrative imaging data, the field generator(s) may be marked or tagged with one or more marker patterns. The marker pattern may be designed such that field generator can be recognized and distinguished from one another, and so that their three-dimensional orientation can be assessed.

In yet another example, the non-invasive imaging data comprises only non-penetrative imaging data. The one or more anatomical structures of the subject may be identified, for instance, by determining an outer body shape of the subject (from the non-penetrative imaging data) and using a body model to identify or predict locations or positions of the anatomical structure(s) inside the subject. The body model may, for instance, identify predicted positions for any anatomical structures within a generic body shape. The determined outer body shape may be mapped to the generic body shape, which mapping is then used to predict the location(s) of any anatomical structures.

The processing unit 520 is further configured to generate feedback information 535 responsive to the determined spatial relationship.

The feedback information may comprise calibration information for an anatomical mapping system and/or location tracking system that uses the one or more electric, magnetic and/or electromagnetic fields generated using the one or more field generators to perform an anatomical mapping or location tracking operation respectively.

For instance, the calibration information may comprise the determined spatial relationship between the one or more anatomical structures of the subject and the one or more field generators. This spatial relationship provides useful a-priori knowledge for improving the accuracy of a mapping or transfer function during a later mapping or locating technique and/or for gating when samples of responses/measurements of any field-sensitive sensor is obtained and/or for defining which responses/measurements of any field-sensitive sensor are used to generate or utilize the mapping/transfer function.

In some examples, the processing unit is further configured to process the non-invasive imaging data to identify a size and/or shape of the one or more anatomical structures. The calibration information may further include information on the identified size and/or shape of the one or more anatomical structures. The identified size and shape provides useful information for assessing predicted absorption or attenuation of fields, to thereby improve the accuracy of a mapping or transfer function.

In at least one example, the calibration information comprises time-dependent spatial information. In this way, the spatial information may effectively temporally track the spatial relationship between the field generators and the one or more anatomical structures. In such examples, the calibration information could be used to select or filter responses or measurements obtained by any field-responsive sensor or control when such responses and/or measurements are sampled.

More specifically, the time-dependent spatial information may be used so that only responses/measurements sampled when the spatial information meets some predetermined criterion/criteria are used to generate a mapping/transfer function and/or processed by a mapping/transfer function.

In some examples, the time-dependent spatial information may be used so that only responses/measurements sampled when the spatial information meets some predetermined criterion/criteria are used to generate the anatomical model and/or to track the location of a sensor and/or device comprising the sensor.

Alternatively and/or additionally, the feedback information may comprise repositioning information indicating any recommended positional changes for the one or more field generators to realize a desired spatial relationship between the one or more anatomical structures of the subject and the one or more field generators.

In a preferred embodiment, the repositioning information provides recommended field generator positions or recommended changes to the field generator positions to achieve the recommended field generator positions, e.g. information on how to move the field generators to the recommended field generator positions.

Recommended changes to the field generator positions may include one or more indicators on how to move one or more of the field generators to recommended field generator positions.

It is preferred for any fields generated by the field generator(s) to overlap or intersect at a desired region for investigation (e.g. to overlap or intersect at a desired anatomical structure for investigation). Accordingly, the recommended field generator positions may be such that, when at the recommended field generator positions, fields generated by the field generators intersect at a desired anatomical structure (being one of the identified one or more anatomical structures).

In other words, the recommended field generator position may be such that, when at the recommended field generator positions, a region of interest (e.g. a desired anatomical structure) would be intersected by fields generated by the field generators or field generator pairs. In preferable examples, this region of interest may lie in (e.g. exactly the) middle of these fields.

This region of interest may represent an area to be investigated by an interventional device having one or more field-sensitive sensors, to facilitate accurate tracking of the sensor(s) and/or the interventional device. In other words, wherein the region of interest may comprise at least one desired anatomical structure for insertion of an interventional device.

The region of interest may be one of the anatomical structures identified by the processing unit 520.

In some examples, the recommended field generator positions may be such that, when at the recommended field generator positions, the field generators or field generator pairs are located orthogonally (i.e., fully perpendicular) with respect to each other.

In some examples, the recommended field generator positions may be such that any fields generated by the field generators or field generator pairs are substantially orthogonal (i.e., fully perpendicular) with respect to each other at the desired anatomical structure. This embodiment would provide more uniform fields and therefore reduce an error in mapping a sample of a field sensitive sensor to a position in space.

Additionally or alternatively, the recommended field generator position may be such that, when at the recommended field generator positions, any fields generated by the field generators or field generator pairs avoid one or more anatomical structures that would distort the field(s). Suitable example include any ill-conducting anatomical structures, such as the lungs (e.g. due to the presence of air in the lungs) and other anatomical structures. This approach improves the uniformity of the fields and thereby the accuracy of tracking approaches that make use of such a field.

It can be generally assumed that the more distortion in any generated fields are distorted, the larger the error will be in mapping from measurements of field-sensitive sensors to position. In one embodiment, for each of a plurality of potential field generator positions, simulation software could process the identified one or more anatomical structures to predict the properties of fields in the subject when said fields are generated by the field generators at the potential field generator positions. For instance, it is possible to determine generated fields near a desired anatomical structure (e.g. the heart) when information about any surrounding tissue or other anatomical structures (such as the lungs, esophagus etc.) is known. By performing such a simulation for multiple potential field generator positions, it is therefore possible to identify the set of potential field generator positions for which the fields in the desired anatomical structure are least distorted (amongst the tested sets). This set of potential field generator positions could be used as the recommended field generator positions.

The output interface 530, if present, is configured to provide the feedback information 535. For instance, the output interface may provide the feedback information to a user interface 570 that provides a user-perceptible output responsive to the feedback information. In another example, the output interface may provide the feedback information 535 to a further processing system 580 for further processing.

If the feedback information includes repositioning information, the output interface controls the user interface to provide guidance for field generator placement. This may be performed by way of one or more (visual) indicators on a screen or display. Alternatively, optical markers (e.g. a laser or light shining directly on the body) or augmented reality (e.g. by means of glasses) visual indicators could be used.

Fig. 6 illustrates a system 600 in which the processing system 500 of Fig. 5 can be employed.

The system 600 thereby comprises the processing system 500 previously described.

The system 600 comprises a surgical navigation system 610. The surgical navigation system 610 comprises a penetrative imaging system 611 and a non-penetrative imaging system 612, both configured for imaging a subject 690.

The penetrative imaging system 611 comprises an emitter arrangement 611A and a detector arrangement 611B. The emitter arrangement 611A emit penetrative waves 611C that are partially absorbed by anatomical features of the subject 690 before the partially absorbed penetrative waves are sensed by the detector arrangement 611B. The response of the detector arrangement to the partially absorbed penetrative waves is processed to generate penetrative imaging data of the subject. Suitable examples of penetrative waves include ionizing radiation, such as X-ray radiation, and non-ionizing radiation, such as ultrasound and/or magnetic fields or radio waves (e.g. for use in magnetic resonance imaging).

The non-penetrative imaging system 612 comprises at least a detector system 612B configured to detect non-penetrative waves reflected by the subject 690, such as light or infrared waves. The detector system may, for instance, comprise a camera. The response of the detector system to the non-penetrative waves reflected from the subject is processed to generate non-penetrative imaging data of the subject. Optionally, the non-penetrative imaging system 612 may also comprise an emitter system (e.g. a light source) for illuminating the subject with non-penetrative waves.

The penetrative imaging data and the non-penetrative imaging data may together form the non-invasive imaging data used by the processing system. In particular, the penetrative imaging data may contain the representation of the one or more anatomical structures and the non-penetrative imaging data may contain the representation of the field generator(s).

The detector arrangement of the penetrative imaging system and the detector system of the non-penetrative imaging system may have a known spatial relationship with respect to one another. This improves an ease and accuracy of defining a co-ordinate system to be shared by the penetrative imaging data and the non-penetrative imaging data, i.e. in registering the two sets of imaging data together. This approach thereby improves an accuracy in identifying a spatial relationship between any field generator(s) and the anatomical structure(s).

The system 600 may comprise an anatomical mapping system 620. The anatomical mapping system 620 and the processing system 500 may together form a mapping system according to an embodiment.

The anatomical mapping system comprises 620 an input module 621 configured to receive: one or more electrical responses, each electrical response being a response of a field-sensitive sensor, positioned on or in an interventional device 629, to any electric, magnetic and/or electromagnetic fields generated by the one or more field generators as the interventional device moves within an anatomical cavity of the subject; and the feedback information generated by the processing unit of the processing system.

The anatomical mapping system may further comprise a processing module 622, communicatively coupled to the input module, configured to process the one or more electrical responses to generate an anatomical map or model of the anatomical cavity.

The processing module is configured to calibrate the one or more electrical responses using the calibration information.

General approaches for generating an anatomical map from the response(s) or measurement(s) taken by one or more field-sensitive sensors have been previously described, with particular reference to Fig. 3. Generally, this approach comprises firstly defining a mapping or transfer function that maps sampled/captured responses or measurements to locations/positions in a co-ordinate space. Determined locations of the sensor(s) and/or the device containing the sensor(s) represent areas within an anatomical element that have been explored by the device. Determined locations can thereby be then processed to generate an anatomical model of an anatomical element.

Mapping or transfer functions used in such approaches, to map a response to a location in space, can be appropriately generated, calibrated or otherwise modified based on calibration information provided by the processing system 500. Approaches for generating a mapping function using responses/measurements have been previously described.

In particular examples, the mapping function is generated using only those responses or measurements of the field-sensitive sensor(s) that were sampled when the spatial relationship between the field generator(s) and the anatomical element(s) meets some predetermined criterion/criteria. Effectively, this means that the responses or measurements may be gated based on the spatial relationship between the between the field generator(s) and the anatomical element(s).

The predetermined criterion/criteria may include a criterion that the spatial relationship matches (e.g. within a predetermined error margin) a predefined spatial relationship. The predetermined error margin may be ±1%, ±5% or ±10%, although other suitable margins will be apparent to the skilled person.

In some examples, the spatial relationship indicates one or more measures of position and/or (difference in) orientation between each field generator and each anatomical structure. The predetermined criterion/criteria may be considered to match (e.g. within a predetermined error margin) a predefined spatial relationship if each of these one or more measures match corresponding predetermined or predefined measures (e.g. within a predetermined error margin) or if at least a predetermined proportion/percentage of these measures match corresponding predetermined or predefined measures (e.g. within a predetermined error margin).

This predefined spatial relationship may be user-defined (e.g. a user marking when a spatial relationship is most appropriate) or automatically determined. An automatically determined spatial relationship may be one in which it is predicted that the field(s) generated by the field generator(s) are homogenous or at a (local) maximum homogeneity for the subject. As another example, a predefined spatial relationship may simply represent a spatial relationship when a first sample of the response/measurement of the sensor(s) is taken (e.g. responsive to a user trigger or the like), so that subsequent sampling is performed with the field generators at a same relative position for improved accuracy in the mapping.

In this way, the calibration information may comprise time-dependent spatial information. Each response/measurement may be associated with a particular sampling time. In an embodiment, only those responses/measurements associated with a sampling time for which the corresponding (time-dependent) spatial information meets some predetermined criterion/criteria are used to generate the mapping/transfer function.

In other examples, the calibration information may be used to controlling sampling apparatus, so that responses/measurements by the sensor(s) are only sampled when the spatial information meets some predetermined criterion/criteria.

Similarly, in subsequent use of the mapping/transfer function, embodiments may be configured such that the only responses/measurements used to determine locations are those sampled by the sensor(s) when the spatial relationship between the field generator(s) and the anatomical element(s) meets the predetermined criterion/criteria. This may comprise controlling when responses/measurements are sampled or filtering/selecting sampled responses/measurements.

The anatomical mapping system 620 may further comprise an output module 623 configured to provide the anatomical map or model. For instance, the output module may provide the anatomical map/model to a user interface 670 that provides a user-perceptible output responsive to the anatomical map/model. In another example, the output interface may provide the anatomical map/mode to a further processing system 680.

The system 600 may comprise the user interface 670.

In some examples, the combination of the user interface 670 and the processing system forms a repositioning feedback system. In this example, the feedback information may comprise the repositioning information, and the user interface may be configured to provide a visual representation of the repositioning information.

The user interface 670 may, as illustrated, comprise a display that is configured to provide one or more visual indicators for repositioning a respective one or more field generators. These indicators may be in the form of arrows or the like superimposed upon a representation of the subject's body.

However, alternative examples are also envisaged. For instance, the user interface may comprise a directable light or laser that can provide one or more optical markers for recommended positions. In an alternative, the user interface may comprise a set of glasses or other augmented reality provision device that is able to provide visual indicators of recommended changes (to achieve recommended field generator positions) via augmented reality.

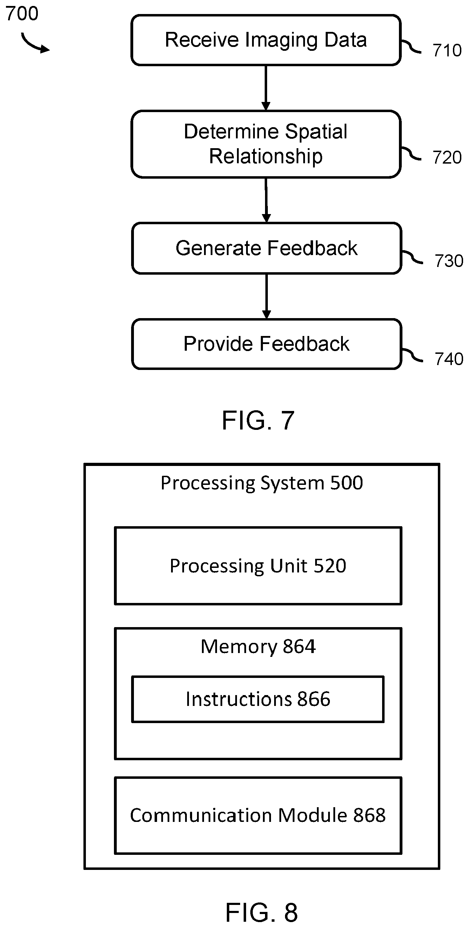

Fig. 7 illustrates a computer-implemented method 700 for aiding an analysis of an anatomical element of a subject. The method may, for instance, be performed by a processing system.

The computer-implemented method 700 comprises a step 710 of receiving, at an input interface non-invasive imaging data of a subject, wherein the non-invasive imaging data contains a representation of one or more anatomical structures of the subject and the one or more field generators positioned on the surface of the subject.

The non-invasive imaging data is generated through a non-invasive imaging procedure of the subject and the one or more field generators are usable for generating one or more electric, magnetic and/or electromagnetic fields for tracking the location of an interventional device in the subject.

The non-invasive imaging data may, for instance, be obtained from an imaging system or memory/storage unit, which may in turn receive or store data from the imaging system.

The method 700 further comprises a step 720 of processing, using a processing unit, the non-invasive imaging data to determine a spatial relationship between the one or more anatomical structures of the subject and the one or more field generators.

The method 700 further comprises a step 730 of generating, using the processing unit, the feedback information responsive to the determined spatial relationship. The feedback information comprises calibration information and/or repositioning information.

The calibration information is configured for an anatomical mapping system and/or location tracking system that uses the one or more electric, magnetic and/or electromagnetic fields generated using the one or more field generators to perform an anatomical mapping or location tracking operation respectively.

The repositioning information indicates any recommended positional changes for the one or more field generators to realize a desired spatial relationship between the one or more anatomical structures of the subject and the one or more field generators.

The method 700 may further comprise a step 740 of providing, at an output interface, the feedback information. Step 740 may, for instance, comprise providing the feedback information to a user interface that provides a user-perceptible output responsive to the feedback information. In another example, step 740 comprises providing the feedback information to a further processing system for further processing.

Fig. 8 is a schematic diagram of a processing system 500, according to embodiments of the present disclosure. The processing system 500 is configured to carry out a method according to an embodiment, such as the method 700 described with reference to Fig. 5.

As shown, the processing system 500 may include a (data) processing unit 520, a memory 864, and a communication module 868. These elements may be in direct or indirect communication with each other, for example via one or more buses.

The processing unit 520 may include a central processing unit (CPU), a digital signal processor (DSP), an ASIC, a controller, an FPGA, another hardware device, a firmware device, or any combination thereof configured to perform the operations described herein. The processing unit 520 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the processing unit 520), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the processing system 500, or one or more components of the processing system 500, particularly the processing unit 520, to perform the operations described herein. For example, the processing system 500 can execute operations of the method 700.

Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 500, the penetration device and/or the user interface (or other further device). In that regard, the communication module 868 can be an input/output (I/O) device. In some instances, the communication module 868 facilitates direct or indirect communication between various elements of the processing system 500 and/or the processing system (Fig. 9).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figs.. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. A processing system (500) for generating feedback information (535) responsive to positions of one or more field generators (101, 102, 103, 104, 105, 106, 107) on the surface of a subject (190, 305, 690), wherein the one or more field generators are usable for generating one or more electric, magnetic and/or electromagnetic fields for tracking the location of an interventional device in the subject, the processing system comprising:
an input interface (510) configured to receive:
non-invasive imaging data (515) of a subject, wherein the non-invasive imaging data contains a representation of one or more anatomical structures of the subject and the one or more field generators positioned on the surface of the subject,
wherein the non-invasive imaging data is generated through a non-invasive imaging procedure of the subject and the one or more field generators;
a processing unit (520) communicatively coupled to the input interface and configured to:
process (720) the non-invasive imaging data to determine a spatial relationship between the one or more anatomical structures of the subject and the one or more field generators; and
generate (730) the feedback information responsive to the determined spatial relationship, wherein the feedback information comprises:
calibration information for an anatomical mapping system and/or location tracking system that uses the one or more electric, magnetic and/or electromagnetic fields generated using the one or more field generators to perform an anatomical mapping or location tracking operation respectively; and/or
repositioning information indicating any recommended positional changes for the one or more field generators to realize a desired spatial relationship between the one or more anatomical structures of the subject and the one or more field generators;
and optionally an output interface (530) configured to provide (740) the feedback information.

2. The processing system of claim 1, wherein the non-invasive imaging data comprises penetrative imaging data of the subject, for identifying the one or more anatomical structures, and non-penetrative imaging data of a surface of the subject, for identifying the one or more field generators.

3. The processing system of any of claims 1 or 2, wherein the non-invasive imaging data is generated by an imaging system that generates imaging data using two or more cameras or detectors having a known spatial relationship with respect to one another.

4. The processing system of any of claims 1 to 3, wherein the one or more anatomical structures of the object comprise one or more anatomical structures that would distort any electric, magnetic and/or electromagnetic fields generated by the one or more field generators.

5. The processing system of any of claims 1 to 4, wherein the one or more anatomical structures comprises at least one desired anatomical structure for insertion of an interventional device.

6. The processing system of claim 5, wherein the feedback information contains repositioning information that identifies recommended changes in position for the one or more field generators such that, when repositioned, an electric, magnetic and/or electromagnetic fields generated by the one or more field generators would intersect the at least one desired anatomical structure.

7. The processing system of any of claims 1 to 6, wherein the calibration information comprises the determined spatial relationship between the one or more anatomical structures of the subject and the one or more field generators.

8. The processing system of claim 7, wherein:
the processing unit is further configured to process the non-invasive imaging data to identify a size and/or shape of the one or more anatomical structures; and
the calibration information further includes information on the identified size and/or shape of the one or more anatomical structures.

9. A mapping system comprising:
the processing system of claim 7 or 8; and
an anatomical mapping system comprising:
an input module configured to receive:
one or more electrical responses, each electrical response being a response of a field-sensitive sensor, positioned on or in an interventional device, to any electric, magnetic and/or electromagnetic fields generated by the one or more field generators as the interventional device moves within an anatomical cavity of the subject; and
the feedback information generated by the processing unit of the processing system;
a processing module, communicatively coupled to the input module, configured to process the one or more electrical responses to generate an anatomical map or model of the anatomical cavity,
wherein the processing module calibrates the generation of the anatomical map or model of the anatomical mapping using the spatial relationship between the one or more anatomical structures of the subject and the one or more field generators contained in the feedback information; and
optionally, an output module configured to provide the anatomical map or model.

10. The system of claim 9, wherein each electrical response is a voltage measured by a voltage sensor positioned on or in the interventional device, and the processing module is configured to generate the anatomical map or model of the anatomical cavity using a voltage-to-position function.

11. The system of claim 9 or 10, wherein the processing module of the anatomical mapping system is configured to calibrate the generation of the anatomical model by, in generating the anatomical model, only processing electrical responses that were sampled at a time when the spatial relationship between the one or more anatomical structures of the subject and the one or more field generators contained in the feedback information met one or more predetermined criteria.

12. The system of claim 11, wherein the one or more predetermined criteria include a criterion that the spatial relationship matches, optionally within a predetermined error margin, a predefined spatial relationship

13. A repositioning feedback system comprising:
the processing system of any of claims 1 to 8, wherein the feedback information comprises the repositioning information; and
a user interface configured to display a visual representation of the repositioning information.

14. A computer-implemented method (700) for generating feedback information (535) responsive to positions of one or more field generators (101, 102, 103, 104, 105, 106, 107) on the surface of a subject (190, 305, 690), wherein the one or more field generators are usable for generating one or more electric, magnetic and/or electromagnetic fields for tracking the location of an interventional device in the subject, the computer-implemented method comprising:
receiving (710), at an input interface (510):
non-invasive imaging data of a subject, wherein the non-invasive imaging data contains a representation of one or more anatomical structures of the subject and the one or more field generators positioned on the surface of the subject,
wherein the non-invasive imaging data is generated through a non-invasive imaging procedure of the subject;
processing (720), using a processing unit (520), the non-invasive imaging data to determine a spatial relationship between the one or more anatomical structures of the subject and the one or more field generators; and
generating (730), using the processing unit, the feedback information responsive to the determined spatial relationship, wherein the feedback information comprises:
calibration information for an anatomical mapping system and/or location tracking system that uses the one or more electric, magnetic and/or electromagnetic fields generated using the one or more field generators to perform an anatomical mapping or location tracking operation respectively; and/or
repositioning information indicating any recommended positional changes for the one or more field generators to realize a desired spatial relationship between the one or more anatomical structures of the subject and the one or more field generators;
and optionally providing (740), at an output interface, the feedback information.

15. A computer program product comprising computer program code means which, when executed on a computing device having a processing system, cause the processing system to perform all of the steps of the method of claim 14.
